# EUROPÄISCHE PATENTSCHRIFT

(11) **EP 0 358 728 B1**
(45) Veröffentlichungstag und Bekanntmachung des Hinweises auf die Patenterteilung: **09.03.1994**
(21) Anmeldenummer: 89900579.7
(22) Anmeldetag: 22.12.1988
(51) Int. Cl.: A61K 7/16

(54) **VORRICHTUNG ZUR BESEITIGUNG VON ZAHNLÄSIONEN**
DEVICE FOR ELIMINATING DENTAL LESIONS
DISPOSITIF D'ELIMINATION DE LESIONS DENTAIRES

(30) Priorität: 23.12.1987 DE 3743719; 03.12.1988 DE 3840860
(43) Veröffentlichungstag der Anmeldung: 21.03.1990
(73) Patentinhaber: BEGO Bremer Goldschlägerei Wilh. Herbst GmbH & Co., 28329 Bremen (DE)
(72) Erfinder: Wiedemann, Wolfgang, Dr., Prof., 97204 Höchberg (DE)
(74) Vertreter: Möller, Friedrich, Dipl.-Ing.
(86) Internationale Anmeldenummer: DE8800778
(87) Internationale Veröffentlichungsnummer: WO8905628

(56) Entgegenhaltungen:
- EP-A- 0 137 436
- DE-A- 2 643 305
- FR-A- 1 293 516
- FR-A- 2 202 697
- FR-A- 2 383 661
- GB-A- 1 212 242
- CARIES RESEARCH, Vol. 19, no. 3, 1985, F.F. Feagin et al.: "Chemical and physical evaluation of dialysed-reconstituted acidified gelatin surface lesions of human enamel", pages 219-227

## Beschreibung

Die Erfindung bezieht sich auf eine Vorrichtung zur Beseitigung von Zahnläsionen.

Bekannt ist, daß bei bestimmten häufig auftretenden Erkrankungen, wie der Karies, Läsionen durch punktuelle Zerstörung des Zahnschmelzes und mitunter auch des darunter befindlichen Dentins entstehen. Als präparative Maβnahme zur Erhaltung des Zahnes geht der Zahnarzt wie folgt vor: Zunächst wird der beschädigte Bereich des Zahnschmelzes und Dentins mit Hilfe eines Bohrers entfernt, so daß eine Kavität entsteht. Nach einer Desinfektion erfolgt anschließend deren Ausfüllen. Des eingesetzte Material in Form von Amalgan, Kunststoff, Zement, Gold oder dgl. wird, um ein Eindringen von Schmutz, Bakterien oder ähnlichem zu verhindern, weitgehend undurchlässig gewählt. Zum Schluß wird die Oberfläche des Füllmaterials und insbesondere auch die Übergangszone poliert, um ein erneutes Entstehen der Karies zu unterbinden.

In der FR-A-2 202 697 werden Zahnpflegemittel beschrieben, welche aufeinander folgend kationische und anionische Komponenten in die Mundhöhle abgeben. Jede Lösung kann dabei einen pH-Wert von etwa 3 - 10 haben.

Die FR-A-1 293 516 betrifft ein Reinigungsmittel für Zähne. Dieses verfügt über eine süße und eine saure Substanz. Dadurch soll der Speichelfluß angeregt werden, wodurch Kohlehydratrückstände von den Zähnen abgespült werden, worauf der Zahnreinigungseffekt beruht.

Hiervon ausgehend hat sich die Erfindung die Schaffung einer Vorrichtung zur Aufgabe gemacht, mit deren Hilfe Zahnläsionen ohne unmittelbaren Eingriff beseitigbar sind.

Gelöst wird diese Aufgabe durch eine Vesrichtung gemäß Anspruch 1.

Der Grundgedanke der Erfindung besteht darin, in der Mundflora, also insbesondere dem Speichel, einen Wechsel zwischen sauerem und alkalischem pH-Wert zu erzeugen, in dem entweder sauere oder alkalische Substanzen additiv beigemischt oder aber solche Substanzen im Wechsel zugefügt werden, die entweder einen saueren oder alkalischen pH-Wert erzeugen. Entscheidend ist das Ergebnis, nämlich die Entstehung sauerer oder alkalischer Verhältnisse.

Die Erfindung geht davon aus, daß kleinere, beispielsweise durch säurehaltige Speisen oder durch kleinere Verletzungen verursachte Zahnläsionen zunächst durch oberflächliche Ablösung des den Zahnschmelz bildenden Calziumphosphates entstehen, die jedoch reversibel sind und rückgängig gemacht werden können. Deren Beseitigung geschieht durch Mineralisation, d.h. durch Niederschlag und kristallinem Einhau von Calziumphosphat. Im Speichel der Mundhöhle befinden sich Calzium- und Phosphationen in wechselndem u.a. durch die aufgenommene Nahrung bestimmtem Anteil. Für die Mineralisation, d.h. die Wiederherstellung des Zahnschmelzes ist gemäß der Lehre der Erfindung der pH-Wert und insbesondere dessen Änderung von entscheidender Bedeutung. Bei niedrigen pH-Wert geht ein großer Teil des Calziumphosphates in Lösung, bei hohem pH-Wert, hingegen fällt das Calziumphosphat aus. Zur Unterstützung der Remineralisation des Zuhnschmelzes ist der Wechsel des pH-Wertes von größer Wichtigkeit, wobei der niedrige pH-Wert erforderlich ist, um eine hinreichend hohe Konzentration an Calziumphosphationen bis zur notwendigen Tiefe eindiffundieren zu lassen. Der hohe pH-Wert bedingt die Ausfällung des Calziumphosphates, d.h. den Niederschlag und die Mineralisation, da andernfalls das Calziumphosphat zwar eindiffundieren, jedoch nicht in hinreichender Menge eingebaut werden würde.

Die erfindungsgemäße Vorrichtung ist als klinisch anwendbares Gerät vorstellbar, mit dessen Hilfe auf definierte Art und Weise der pH-Wert beeinflußt und verändert wird, in dem die entsprechenden, vorgenannten Substanzen, worunter sowohl Feststoffe als auch Flüssigkeiten zu verstehen sind, eingebracht werden. Unabhängig von der klinischen Anwendring sind auch ambulante Einsätze durch vorübergehendes Implantieren einer erfindungsgemäßen Vorrichtung denkbar.

Der entscheidende Vorteil der erfindungsgemäßen Vorrichtung ist darin zu sehen, daß ohne unmittelbaren Eingriff eine Beseitigung von Zahnläsionen und erneute Entstehung des aus Calziumphosphat aufgebauten Zahnschmelzes erfolgt. Die erfindungsgemäße Vorrichtung ist in erster Linie zur Beseitigung von Zahnläsionen geringeren Ausmaßes geeignet. Darüber hinaus ist sie jedoch auch bei größeren Läsionen dann einsetzbar, wenn auch der größere beschädigte Bereich des Zahnschmelzes mit einer Einlage aus entsprechendem Material, z.B. Apatit versehen und dann erst das erfindungsgemäße Gerät eingesetzt wird. Des weiteren ist ein Einsatz im proghylaktischen Sinne zur Härtung des Zahnschmelzes denkbar und empfehlenswert.

Genauere Untersuchungen haben gezeigt, daß ein sägezahnförmiger Verlauf des pH-Wertes in Abhängigkeit von der Zeit ideal ist, d.h. der Anstieg des pH-Wertes soll vergleichsweise langsam von statten gehen, der Abfall demgegenüber jedoch wesentlich schneller erfolgen. Bei einer zu schnellen Erhöhung des pH-Wertes entsteht sehr rasch an der Grenzfläche des Zahnes durch Mineralisation eine undurchlässige Trennschicht, die das weitere Eindiffundieren unterbindet. Ein sehr langsamer pH-Wert-Anstieg bedeutet hingegen ein Ausspülen und Beseitigen von bereits eindiffundierten, jedoch noch gelösten Calziumphosphaten. Zur Realisierung des langsamen Anstieges des pH-Wertes empfiehlt sich der Einsatz von Substanzen in festem Aggregatszustand, die langsam vom Speichel aufgelöst wird. Demgegenüber ist zur Erreichung eines schnellen Abfalls das Einbringen von Lösungen oder Flüssigkeiten bevorzugt, da sie sich schneller verteilen und ihre Wirksamkeit entfalten.

Die konkrete Ausbildung der vorgeschlagenen Vorrichtung ist in weiten Grenzen beliebig und sie kann beispielsweise als Gerät erfolgen, welches über einen Schlauch und ein Mundstück die Beeinflussung des pH-Wertes der Mundflora Vornimmt. Über einen vergleichsweise kurzen Zeitraum lassen sieh dann ideale Verhältnisse herstellen. Auch wäre die vorübergehende Aufnahme einer hinreichend kleinen Vorrichtung im Mundraum eine mögliche Lösung.

Eine demgegenüber von der Handhabung besonders elegante Lösung besteht in der Verwendung einer Solvette (= Lutschbonbon), welches aus mehreren alternierend aus saueren oder alkalischen bzw. aus saueren oder alkalischen pH-Wert erzeugenden Substanzen schalenförmig aufgebaut ist. Die Solvette löst sich in der üblichen Weise von außen nach innen zu auf, so daß sich wechselnde Verhältnisse des pH-Wertes einstellen. Über die Dicke der jeweiligen Schale ist eine Einflußnahme auf die Zeitabhängigkeit des pH-Wert-Wechsels möglich.

Wie bereits ausgeführt, geht in sauerem Zustand (niedriger pH-Wert) sehr viel Calziumphoshat in Lösung. Nachdem nicht sicher ist, daß beim sehr raschen Herabsetzen des pH-Wertes auf einen niedrigen Wert, die dann in Lösung gehenden Calziumphosphationen sofort auf natürliche Weise zur Verfügung gestellt werden, empfiehlt sich in zweckmäßiger Weiterbildung die die niedrigen pH-Werte erzeugenden Schalen bzw. die dementsprechenden Substanzen mit Calzium- und Phosphatanteilen anzureichern, so daß eine Zurverfügungstellung auf natürliche Weise nicht in vollem Umfang erforderlich ist. Insbesondere ist ausgesechlossen, daß sich ein Teil des mineralisierten Calziumphosphates an der Zahnoberfläche in Lösung geht.

Als den pH-Wert in die Richtung auf eine Säure hin verändernde, also der Senkung des pH-Wertes dienende Substanz werden vor allem organische Säuren wie z.B. Milchsäuren als besonders geeignet angesehen. In der Handhabung sind sie unproblematisch und für den Patienten ungefährlich.

Ähnliches gilt bei Verwendung von Calzium- und Na-lactat zur Herstellung des alkalischen Zustandes.

Praktische Versuche haben gezeigt, daß es mitunter zum Auftreten unerwünschter Regelschwingungen kommen kann. Zu deren Unterdrückung und zur Stabilisierung der Diffusions- und Mineralisationsvorgänge hat die Erfindung die Beigabe von Fluorid als besonders geeignet erkannt.

Die Wirkung der soeben aufgezählten Substanzen besteht darin, daß sie den pH-Wert der Mundflora durch additives Beimischen in der gewünschten Weise verändern. Ein hiervon völlig anders gestalteter Weg besteht in der Beigabe von Substanzen, die aus sieh heraus weder alkalisch noch sauer sind. Statt dessen wirken sie auf die zahlreichen im Plaque befindlichen Bakterien in dem Sinne ein, daß sie sauere oder basische Stoffwechselprodukte erzeugen und ausscheiden. Man macht sich die Erkenntnis zu Nutze, daß bei Vorliegen bestimmter Substanzen die Bakterien überwiegend sauere oder alkalische Stoffwechselprodukte erzeugen. Der Vorteil besteht darin, daß der pH-Wert genau dort, wo sich pH-Wert in erster Linie ändern muß, nämlich auf der Oberfläche des Zahnes, durch die dort befindlichen Bakterien verändert wird.

Wird der Übergang in den saueren Bereich gewünscht, wird die Einnahme zuckerhaltiger Verbindungen empfohlen, da dann die Bakterien sauere Stoffwechselprodukte erzeugen.

Werden hingegen basische Verhältnisse erwünscht, ist die Einnahme eiweißhaltiger Substanzen von Vorteil, da dann die Bakterien basische Stoffwechselprodukte zur Unterstützung der Mineralisation zur Verfügung stellen. Entscheidend ist, daß die Stoffwechselprodukte genau dort anfallen, wo sie auch benötigt werden.

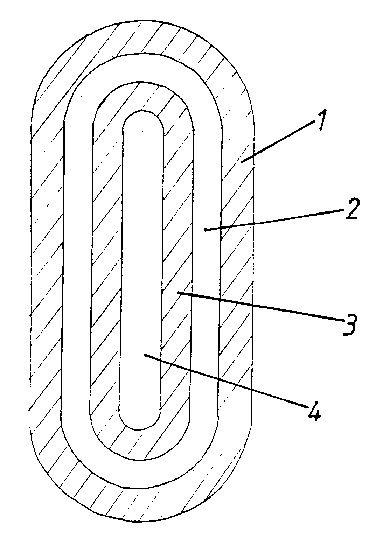

Weitere Einzelheiten, Merkmale und Vorteile der Erfindung lassen sich dem nachfolgenden Beschreibungsteil entnehmen, indem anhand der Zeichnung ein Ausführungsbeispiel der Erfindung näher erläutert wird. Sie zeigt in schematisch gehaltener Querschnittsdarstellung eine Solvette gemäß der Erfindung.

Sie besteht aus insgesamt 4 Schalen (1 - 4), von denen die äußerste sowie die dritte, also vorletzte Schalen (1,3) von gleicher substanzlicher Beschaffenheit, also z.B. alkalisch sind und deshalb auf gleiche Weise in schraffierter Darstellung gehalten wurden. Die zweite (2) sowie die innerste Schale (4) hingegen sind von entgegengesetztem pH-Wert.

Während des Lutschvorganges wird nach und nach zunächst die äußerste alkalische Schale (1) abgetragen, was einen Anstieg des pH-Wertes bedingt. Mit Beginn der Schale (2) wird der pH-Wert wieder abgesenkt und sauere Verhältnisse geschaffen. Mit beginnenden Abtrag der dritten Schale (3) steigt der pH-Wert wieder an und zwar so lange, bis nurmehr noch die letzte Schale (4) übrigbleibt. Dem vierschichtigen Aufbau der Solvette folgt ein vierfacher Wechsel des pH-Wertes. Durch entsprechende Veränderung der Zahl der einzelnen Schichten (1) bis (4) ließe sich die Anzahl der Wechsel problemlos verändern und beeinflussen sowie durch die Änderung der Dicke der einzelnen Schale (1) bis (4) eine Einflußnahme auf die Zeitkonstante erreicht.

Im Ergebnis erhält man eine Solvette, die in optimaler Weise die Beseitigung von Zahnläsionen im Dentin und die Mineralisation des Zahnschmelzes auf unproblematische Weise gestattet.

## Patentansprüche

1. Vorrichtung zur Beseitigung von Zahnläsionen, insbesondere Solvette, mit mehreren Substanzen, dadurch gekennzeichnet, daß die Substanzen in unterschiedlichen Schalen (1 bis 4) angeordnet sind, die im zyklischen Wechsel einen sauren und alkalischen pH-Wert in der Mundhöhle erzeugen oder an den Speichel abgeben.

2. Vorrichtung nach Anspruch 1, dadurch gekennzeichnet, daß die Substanzen derart sind, daß ein sägezahnförmiger Verlauf des pH-Werts entsteht.

3. Vorrichtung nach Anspruch 1 oder 2, dadurch gekennzeichnet, daß mindestens eine der Schalen (1 bis 4) einen radialen Konzentrationsgradienten aufweist.

4. Vorrichtung nach Anspruch 1, dadurch gekennzeichnet, daß die saure Substanz Calzium und/oder Phosphor enthält.

5. Vorrichtung nach Anspruch 1, gekennzeichnet durch organische Säure, zum Beispiel Milchsäure, als saure Substanz.

6. Vorrichtung nach Anspruch 1, gekennzeichnet durch Ca-lactat oder Na-lactat als alkalische Substanz.

7. Vorrichtung nach einem der Ansprüche 1 bis 6, gekennzeichnet durch einen Anteil an Fluorid.

8. Vorrichtung nach einem der Ansprüche 1 bis 7, dadurch gekennzeichnet, daß die einen sauren pH-Wert erzeugende Substanz Zucker ist.

9. Vorrichtung nach einem der Ansprüche 1 bis 8, dadurch gekennzeichnet, daß die einen alkalischen ph-Wert erzeugende Substanz Eiweiß ist.

## Claims

1. Apparatus for the removal of dental lesions, especially a pastille, with a plurality of substances, characterized in that the substances are arranged on different shells (1 to 4), which create, in cyclic alternation, an acidic and alcaline pH value in the mouth cavity or give it off to the saliva.

2. Apparatus according to claim 1, characterized in that the substances are such that the course of the pH value takes the form of a sawtooth.

3. Apparatus according to claim 1 or 2, characterized in that at least one of the shells (1 to 4) has a radial concentration gradient.

4. Apparatus according to claim 1, characterized in that the acidic substance contains clacium and/or phosphorus.

5. Apparatus according to claim 1, characterized by organic acid, for example lactic acid, as an acidic substance.

6. Apparatus according to claim 1, characterized by Ca-lactate or Na-lactate as an alcaline substance.

7. Apparatus according to one of the claims 1 to 6, characterized by a proportion of flouride.

8. Apparatus according to one of the claims 1 to 7, characterized in that sugar is the substance which creates an acidic pH value.

9. Apparatus according to one of the claims 1 to 8, characterized in that protein is the substance which creates an alcaline pH value.

## Revendications

1. Dispositif pour éliminer des lésions dentaires, en particulier bonbon à sucer, comprenant plusieurs substances, caractérisé en ce que les substances sont agencées dans différentes coques (1 à 4) qui, dans une alternance cyclique, produisent dans la cavité buccale une valeur de pH acide et une valeur de pH alcaline ou la fournissent à la salive.

2. Dispositif suivant la revendication 1, caractérisé en ce que les substances sont telles qu'on obtient un parcours en dent de scie de la valeur du pH.

3. Dispositif suivant l'une des revendications 1 et 2, caractérisé en ce qu'au moins une des coques (1 à 4) présente un gradient de concentration radial.

4. Dispositif suivant la revendication 1, caractérisé en ce que la substance acide contient du calcium et/ou du phosphore.

5. Dispositif suivant la revendication 1, caractérisé par de l'acide organique, par exemple de l'acide lactique, comme substance acide.

6. Dispositif suivant la revendication 1, caractérisé par du lactate de Ca ou du lactate de Na, comme substance alcaline.

7. Dispositif suivant l'une des revendications 1 à 6, caractérisé par une fraction de fluorure.

8. Dispositif suivant l'une des revendications 1 à 7, caractérisé en ce que la substance produisant une valeur de pH acide est du sucre.

9. Dispositif suivant l'une des revendications 1 à 8, caractérisé en ce que la substance produisant une valeur de pH alcaline est un protide.
